# EUROPEAN PATENT SPECIFICATION

(11) **EP 2 686 027 B1**
(45) Date of publication and mention of the grant of the patent: **05.05.2021**
(21) Application number: 11708310.5
(22) Date of filing: 16.03.2011
(51) Int. Cl.: A61L 27/12, A61L 27/22, A61L 27/46, A61L 27/54

(54) **PHARMACEUTICAL FORMULATION FOR USE IN SPINAL FUSION**
PHARMAZEUTISCHE FORMULIERUNG ZUR VERWENDUNG FÜR DIE WIRBELSÄULENFUSION
FORMULATION PHARMACEUTIQUE DESTINÉE À ÊTRE UTILISÉE DANS UNE FUSION DES VERTÈBRES

(43) Date of publication of application: 22.01.2014
(73) Proprietor: Kuros Biosurgery AG, 8005 Zürich (CH)
(72) Inventor: SCHENSE, Jason, 8008 Zürich (CH); MARK, Silke, 5200 Brugg (CH); ALVISI, Monica, 8046 Zürich (CH); MARTINEZ VARGAS, Maria Angeles, 8002 Zürich (CH)
(74) Representative: Schmitt-Nilson Schraud Waibel Wohlfrom Patentanwälte Partnerschaft mbB
(86) International application number: PCT/EP2011/054008
(87) International publication number: WO 2012/123028

(56) References cited:
- EP-A1- 1 604 693
- WO-A1-01/48148
- WO-A1-2006/072623
- WO-A2-03/070186
- WO-A2-2006/067221
- WO-A2-2006/072622
- WO-A2-2008/064132
- WO-A2-2009/105723
- WO-A2-2009/120433
- WO-A2-2010/048610
- US-A1- 2003 187 232
- US-A1- 2009 010 940

## Description

Spinal fusion, also known as *spondylodesis* or *spondylosyndesis,* is a surgical treatment method used for the treatment of various morbidities such as degenerative disk disease, spondylolisthesis (slippage of a vertebra), spinal stenosis, scoliosis, fracture, infection or tumor. The aim of the spinal fusion procedure is to reduce instability and thus pain. Patients requiring spinal fusion have either neurological deficits or severe pain which has not responded to conservative treatment. Spinal fusion is achieved by instrumentation to stop movement between the vertebrae and involve removal of the intervertebral disc, laminectomy, roughening of the bone surfaces that are required to be fused and application of a material that stimulates bone growth between adjacent vertebrae. It is estimated that each year only in the US approximately 500,000 spinal fusion procedures are performed.

The spine has three main segments: the cervical spine, the thoracic spine, and the lumbar spine. The cervical spine is the upper part of the spine known as the neck. It is made up of seven vertebrae. The thoracic spine is the center part of the spine. It is made up of 12 vertebrae. The lumbar spine is the lower portion of the spine. It is usually made up of five vertebrae, however, some people may have six lumbar vertebrae. Spinal fusion is done most commonly in the lumbar region of the spine, but it is also used to treat problems in the cervical and, more rarely, thoratic spine.

There are two types of spinal fusion, which may be used either alone or in conjunction with each other:
Posterolateral fusion places the material that stimulates bone growth, i.e. the bone graft or bone graft substitutes, on the spinal gutter in the back of the spine. These vertebrae are then fixed in place with a posterolateral fixation. and the bone bridge is formed between the transverse processes of the spine
Interbody fusion places the bone graft or bone graft subsitute between the vertebra in the area usually occupied by the intervertebral disc. In preparation for the spinal fusion, the disc is removed entirely. A device, the spinal fusion cage, may be placed between the vertebra to maintain spine alignment and disc height. The interbody cage may be made from either synthetic polymers, titanium or other metals or bone. The fusion, i.e bone bridge, occurs between the endplates of the vertebrae. Fusion rates are usually higher with interbody fusion than with poterolateral fusion..

In most cases, the fusion is augmented by a process called fixation, meaning the placement of metallic screws (pedicle screws often made from titanium), rods or plates, to stabilize the vertebra to facilitate bone fusion.

The main challenge in terms of a clinical successful outcome of a spinal fusion procedure is the need to form bone tissue instead of the original tissue in the space between the vertebra. The original tissue in case of interbody fusion was a disc and in case of a posterolateral fusion muscle tissue. As the bone has to grow and replace a different tissue type there is a need to create an appropriate environment to trigger and promote bone formation at all in this area.

Lots of studies have shown that bone graft is needed to create the appropriate environment in order for a solid bone bridge (between the vertebrae) to form. Currently bone graft and a variety of bone graft substitutes are used in spinal fusion with mixed clinical outcome. The bone grafts and bone graft substitutes are autograft (harvested from the iliac crest of the patient's body), allograft, demineralisied bone matrix, various synthetic materials, like calciumphosphates or hydroxyapatitites, stem cell containing products combining stem cells with one of the other classes of bone graft substitutes, and as the latest generation of bone graft substitutes, growth factor containing matrices, such as InFuse™. Autograft is the gold standard in this indication because of efficacy and safety. However, due to limited supply of a patient's own bone, the risk of donor site pain and morbidity (blood loss, infection) in combination with long hospital stays and operation trime, there has been a continued search for ideal bone graft substitutes to replace autologous bone.

The growth factor containing matrices, like InFuse, have demonstrated equivalent fusion rates to autograft and therefore had significant impact on the market, however there are disadvantages associated with this product. Apart from the expensive production process of the BMP (bone morphogenetic protein), the protein is delivered from a collagen matrix in high concentration. Collagen matrices from bovine origin carry all the risks of xenogenic materials, show poor handling properties in the surgical procedure, e.g. are they not moldable to closely fit to the shape of the injury or fusion site, and the high concentration of BMPs delivered to the body can lead to calcification of organs or to bone formation in other parts of the body, so called ectopic bone formation. In particular several complications related to the use of BMP-2 in spinal fusion were reported (e.g neurological complications induced by exuberant bone formation and respiratory complication due to inflammatory response/swelling around the application site). In July 2008 the FDA issued a safety notice relating to the use of InFuse in the cervical spine WO2009/120433 discloses fibrin foams intended for application as wound dressing materials and to fill devices such as spinal fusion cages. Biologically active substances may be added to the fibrin foams, such as PTH.

The object of the present invention is to avoid the drawbacks of the prior art.

It is an object of the present invention to provide a bone graft substitute which effectively fuses vertebrae in a spinal fusion procedures which is safe to use.

In another aspect of the present invention the bone graft substitute is easy to prepare and to apply during the operation procedure, i. e. allows for easy handling.

In still another aspect the bone graft substitute has product characteristics which allow the application of the product during operation without additional damage to the surrounding tissue.

Accordingly, the present invention provides a pharmaceutical formulation for use in a spinal fusion method, a pharmaceutical product for use in a spinal fusion method, an interbody spinal fusion cage comprising the formulation or product, respectively, a kit for use in a spinal fusion method, and a method for forming a pharmaceutical product for use in a spinal fusion method as claimed in the independent claims. Preferred embodiments of the present invention are defined in the respective dependent claims, and are described below. The materials and methods described herein are equally suitable for the pharmaceutical formulation, the pharmaceutical product, and the kit, as well as for use in connection with a spinal fusion cage.

### Summary of the invention

The pharmaceutical formulation for use in spinal fusion procedure comprises (i) a composition for forming a matrix, comprising at least a first matrix material precursor component and a second matrix material precursor component capable of forming the matrix by crosslinking of the precursor components and an bioactive factor, which is biologically active to stimulate bone formation between two vertebrae and for effecting or supporting spinal fusion, the bioactive factor being suitable to be releasably incorporated suitable in the matrix upon crosslinking of the matrix material precursor components, wherein the bioactive factor is PTH. The PTH is present in a concentration range of between 0,01 to 1 mg PTH/mL matrix , preferably of between 0,2 to 0,7 mg PTH/mL matrix, and most preferably of 0,4 mg PTH/mL matrix. In a preferred embodiment the matrix is a fibrin matrix and the composition for forming the fibrin matrix comprises as a first matrix material precursor component fibrinogen and as a second matrix material precursor component thrombin. Optionally one of the precursor components comprises Factor XIIIa and a calcium source. Further, PTH is present in a range of between 0,01 to 1 mg PTH/mL fibrin matrix, preferably in a concentration range of between 0,01 to 1 mg PTH/mL fibrin matrix , more preferably of between 0,2 to 0,7 mg PTH/mL fibrin matrix, and most preferably of 0,4 mg PTH/mL fibrin matrix.

Fibrin matrices can inherently contain very small amounts of BMPs and other growth factors. PTH is the only added peptide or protein factor able to induce bone formation in the formulation.

In a preferred embodiment the PTH is a fusion peptide ("PTH fusion peptide") containing at least two domains wherein the first domain comprises PTH and the second domain comprises a covalently crosslinkable substrate domain able to crosslink to the fibrin matrix during or after its formation. In the present invention, the PTH concentration is always indicated in relation to the matrix material, e. g. the fibrin matrix, irrespective of whether granules are present.

In a preferred embodiment the fibrin matrix contains fibrinogen in a range of 5 to 65 mg per millilitre fibrin matrix, more preferably 15 to 60 mg per millilitre fibrin matrix, even more preferably from 25 to 55 mg per millilitre fibrin matrix, and most preferably 36 to 50 mg per millilitre fibrin matrix. Thrombin is present in a range of 0.5 to 5 I.U. per millilitre fibrin matrix, most preferably 1.25 to 3.25 I.U. per millilitre fibrin matrix. I.U. stands for one international unit of thrombin and is defined as the activity contained in 0.0853 mg of the First International Standard of Human Thrombin.

Additionally a calcium ion source helps to form the fibrin matrix. The calcium ion source is preferably CaCl₂∗ 2H₂O in a concentration of 0.5 to 5 mg per ml fibrin matrix, even more preferable of 2 to 3.5 mg per ml fibrin matrix, most preferably of 2.6 to 3.3 mg per ml fibrin matrix. In one embodiment of the present invention granules, preferably porous calcium containing granules, are present for structural support. If granules are present in the formulation, any water used to wet the granules before mixing is not taken into account for the above mentioned concentration ranges, since said water stays in the pores of the granules throughout the process of matrix formation and thus does not have any dilutive effect on the fibrinogen and thrombin concentration in the fibrin matrix.

Advantageously the PTH is present in a concentration range of between 0,01 to 1 mg PTH/mL of fibrin precursor solutions (i.e. the total of fibrin precursor solutions), preferably of between 0,2 to 0,7 mg/mL fibrin precursor solutions and most preferably of 0,4 mg/mL fibrin precursor solutions

The composition may further contain granular material. The PTH concentration is in the relation to the matrix, irrespective of the presence of granules.

The granular material can be any biocompatible material providing the necessary mechanical support to the pharmaceutical product. Preferably it is a ceramic compound. Biodegradable ceramic compounds have shown favourable properties when used in the PTH composition for the use of the present invention. The ceramic compound preferably comprises a calcium mineral, like hydroxyapatite, calcium phosphate or calcium sulphate. Preferred are biodegradable porous mixtures of hydroxyapatite and tricalciumphosphate, like TricOs from Biomatlante (France) or Camceram from Cam Implants, Leiden (Netherlands). Most preferred is a compound being a mixture of hydroxyapatite (60%) and tricalciumphosphat (40%), marketed under the tradename TricOs. The size of the granules are preferably between 1 and 2 mm.

The ratio of the liquid components in the PTH composition, like fibrinogen and thrombin buffer solution and the liquid to wet the granules, and the granules are regulated by the dead space of the granules. A ratio of 1:1 dead space volume in the granules to total volume of liquids in the PTH composition is the upper limit of granules and constitutes a preferred embodiment of the present invention. Any amounts of granules less than that are possible.

The present invention is furthermore related to a kit comprising the above formulation, wherein at least one of the matrix material precursor components suitable of forming a the matrix is stored separately from the other matrix material precursor components of said matrix.

The aforementioned formulations are used for the manufacture of a pharmaceutical product, i.e. a PTH containing matrix, preferably a PTH containing fibrin or synthetic matrix for the stimulation of spinal fusion, preferably interbody spinal fusion.

When administered locally with appropriate stabilization, the PTH containing matrix, preferably PTH containing fibrin matrix, is capable to stimulate preferably spinal interbody fusion at every level of the spine. Particular preferred indications are cervical and lumbar interbody fusions. The PTH containing fibrin or synthetic matrix can be used in combination with cages of various materials (PEEK, CFRP, titanium etc.). In a preferred embodiment PTH containing fibrin matrix is used with cages made of PEEK or CFRP. PTH containing fibrin matrix can be formed in the cage prior to its implantation or can be formed directly in situ after the cage has been implanted, inside and outside of the cage. In a preferred embodiment, PTH containing fibrin matrix is prepared from the pharmaceutical composition comprising fibrin matrix precursor components and PTH and applied to the cage prior to implantation of the cage and additionally also applied to the disc space prior to insertion of the cage. The pharmaceutical composition comprising the matrix precursor components and PTH can be administered inside and outside of the interbody cage, either in a non-crosslinked, but preferably as a partially crosslinked composition (i.e still moldable) with or without combination with posterior fixation. Additionally a posterolateral fusion procedure can be performed.

Throughout this specification, the present invention is described in particular for a fibrin matrix.It is, however stressed that the invention is equally applicable to any other matrix, e.g synthetic matrices, as described below.

### Detailed description of the invention

It has been surprisingly found that a pharmaceutical product comprising PTH in a matrix demonstrates fusion of adjacent vertebra in a spinal fusion procedure, preferably an interbody spinal fusion procedure Preferably the PTH is releasably incorporated in a matrix and applied to an anatomical location space where bone tissue has to be formed. A preferred location is the disc space and the material is preferably applied in conjunction with an interbody cage. One aspect of the present invention is therefore a pharmaceutical formulation for use in a spinal fusion method comprising a composition for forming a matrix and PTH suitable to be releasably incorporated in the matrix upon formation its formation. The PTH is biologically active to stimulate bone formation between two vertebrae and for effecting or supporting spinal fusion. In another aspect, and not forming part of the present invention, the PTH is loaded into or on an existing matrix, as e.g. a collagen sponge soaked with a PTH solution, and the thus PTH supplemented matrix is implanted into the anatomical location where bone formation is effected. In a preferred embodiment the PTH is bound to a matrix by covalent linkages.

Although locally delivered PTH in fibrin with or without granular material was reported in WO 2006/072622 and WO 2006/072623 to be suitable for the repair of bone fractures, for treatment of SBC (solitary bone cyst) and to increase bone mass in osteoporotic patients, the fact that the pharmaceutical formulation comprising PTH and the pharmaceutical product obtained from the pharmaceutical formulation of the present invention, i.e. locally delivered PTH from a matrix, showed efficacy in a spinal fusion procedure was unexpected for several reasons.

First, each healing environment has unique features, which means that a bone graft substitute must be tested in the specific healing environment anticipated to predict efficicacy. (S. D. Boden, SPINE Volume 27, Number 16S, pp. S26-S31, 2002). This principle is also applied by the FDA. Different healing environments like e.g. metaphyseal defect, long bone fracture, interbody spine fusion have increasing levels of difficulty in forming new bone (Boden S, 2002) and thus validation of any bone graft substitute in one clinical anatomic site is not predictive of its performance in another location (Boden, 2002).

Second, fracture healing is a proliferative physiological process in which the body facilitates the repair of a bone fracture. The body responds to the traumatic event with production of several growth factors in a timely manner. These growth factors are able to attract stem cells and stimulate their proliferation and differentiation into osteoblasts, and ultimately induce bone healing. In contrast, spinal fusion is a surgical procedure intended to achieve bone formation and fusion within two segments of the spine. Therefore there is a need to stimulate *de novo* bone formation.

A material in order to induce *de novo* bone formation must contain one or more growth factors able to induce differentiation of progenitor cells into osteoblasts (Boden 2002). BMPs have the ability to induce de novo bone formation when applied intramuscularly in rat (Hsu, Journal of orthopaedic research, August 2006, pp 1660-1669), therefore their efficacy in spinal fusion was expected. As studies by the inventors have shown, locally delivered PTH in a fibrin matrix does not induce ectopic bone formation, and therefore could not be expected to be able to stimulate interbody spinal fusion. Nevertheless the present inventors could achieve enhanced spinal fusion when presenting PTH in a suitable matrix material allowing controlled release of the PTH incorporated therein, and placing the matrix material having PTH releasably incorporated therein between adjacent vetebrae to be fused.

The matrix is formed by crosslinking ionically covalently, or by combinations thereof at least a first and a second matrix material precursor component and thus forming a three dimensional network, i.e. the matrix or by tailoring natural and/or preformed materials, such as collagen, as the matrix . In one embodiment the matrix is formed of proteins, preferably proteins naturally present in the patient into which the matrix is to be implanted. A particularly preferred matrix of this kind is fibrin, although other proteinacious matrices , such as collagen and gelatine can also be used. Polysaccharides and glycoproteins may also be used as matrix. It is also possible to use synthetic matrix material precursor components which are crosslinkable by ionic or covalent binding to form a matrix. Preferably the matrix is formed by covalent crosslinking of matrix material precursor components.

Generally, matrix materials and matrices as disclosed in WO 00/44808 A1 and WO 03/052 091 A1 are suitable in the present invention.

In a preferred embodiment the matrix is a fibrin matrix. In another preferred embodiment the matrix is a polyalkylene-oxid based synthetic material, preferably a polyethylenoxidbased material.

### Fibrin Matrices and Fibrin Matrix Precursor Components

Fibrin is a natural material which has been reported for several biomedical applications. Matrices made from fibrin have been described as material for cell ingrowth matrices in U.S. Patent No. 6,331,422 to Hubbell et al. Fibrin has been used in sealants because of its ability to bind to many tissues and its natural role in wound healing. Some specific applications include use as a sealant for vascular graft attachment, heart valve attachment Additionally, these matrices have been used as drug delivery devices, and for neuronal regeneration. Although fibrin matrices provide a solid support for tissue regeneration and cell in-growth, there are few active sequences in the monomer that directly enhance these processes.

The process by which fibrinogen is polymerized into fibrin has also been characterized. Initially, a protease cleaves the dimeric fibrinogen molecule at the two symmetric sites. There are several possible proteases than can cleave fibrinogen, including thrombin, peptidase, and protease III, and each one severs the protein at a different site. Once the fibrinogen is cleaved, a self-polymerization step occurs in which the fibrinogen monomers come together and form a non-covalently crosslinked polymer gel. This self-assembly happens because binding sites become exposed after protease cleavage occurs. Once they are exposed, these binding sites in the centre of the molecule can bind to other sites on the fibrinogen chains, which are present at the ends of the peptide chains. In this manner, a polymer network is formed. Factor XIIIa, a transglutaminase activated from Factor XIIIa by thrombin proteolysis, may then covalently crosslink the polymer network. Other transglutaminases exist and may also be involved in covalent crosslinking and grafting to the fibrin network.

Once a crosslinked fibrin matrix is formed, the subsequent degradation is tightly controlled. One of the key molecules in controlling the degradation of fibrin is α2-plasmin inhibitor. This molecule acts by crosslinking to the α chain of fibrin through the action of Factor XIIIa. By attaching itself to the matrix, a high concentration of inhibitor can be localized to the matrix. The inhibitor then acts by preventing the binding of plasminogen to fibrin and inactivating plasmin. The α2-plasmin inhibitor contains a glutamine substrate. The exact sequence has been identified as NQEQVSPL (SEQ ID NO: 12 in the sequence listing in the application WO 03/052 091), with the first glutamine being the active amino acid for crosslinking.

It has been demonstrated in WO 03/ 052 091 that bi-domain peptides, which contain a factor XIIIa substrate sequence and a bioactive peptide sequence, can be crosslinked into a fibrin matrix and that this bioactive peptide retains its cellular activity in *vitro.*

Depending on the indication and substances mixed into the fibrin matrix the concentration of thrombin might vary.

The fibrin matrix precursor components comprise preferably two separate precursor solutions wherein the first fibrin matrix precursor solution contains 10 to 130 mg fibrinogen per millilitre precursor solution, more preferably 30 to 120 mg fibrinogen per millilitre precursor solution, even more preferably from 50 to 110 mg fibrinogen per millilitre precursor solution, and most preferably 72 to 110 mg fibrinogen per millilitre precursor solution. If thrombin has to be added to form the matrix the second fibrin matrix precursor solution contains 1 to 10 I.U. thrombin per millilitre precursor solution , most preferably 2.5 to 6.5 I.U. thrombin per millilitre precursor solution. Additionally a calcium ion source can be in either one of the two precursor solutions. The calcium ion source is preferably CaCl₂ ∗ 2H₂O in a concentration of 1 to 10 mg per ml precursor solution, even more preferably of 4 to 7 mg per ml precursor solution, most preferably of 5.2 to 6.6 mg per ml precursor solution. Optionally, an enzyme capable of catalyzing the matrix formation is added to a precursor solution, like Factor XIIIa. Preferably, Factor XIIIa is present in a concentration of 0.5 to 100 I.U. per millilitre precursor solution, more preferably of 1 to 60 I.U. per millilitre precursor solution, and most preferably of 1 to 10 I.U. per millilitre precursor solution.

Further part of the present invention is the use of a pharmaceutical formulation comprising a composition for forming a fibrin matrix, said composition comprising first and second fibrin matrix precursor components being thrombin and fibrinogen, preferably as a solution, a calcium source and a PTH for the manufacture of a fibrin matrix containing PTH, for the stimulation of spinal fusion, preferably spinal interbody fusion.

### Synthetic matrices and synthetic matrix material precursor components

Crosslinking reactions for forming synthetic matrices for application in the body include (i) free-radical polymerization between two or more matrix material precursors components containing unsaturated double bonds, as described in Hern et al., J. Biomed. Mater. Res. 39:266-276 (1998), (ii) nucleophilic substitution reaction such as e.g. between a precursor component including an amine group and a precursor component including a succinimidyl group as disclosed in U.S. Patent No. 5,874,500 to Rhee et al.*,* (iii) condensation and addition reactions, and (iv) Michael type addition reactions between a matrix material precursor components comprising strong nucleophile and a matrix material precursor components comprising conjugated unsaturated group or bond (as a strong electrophile). Particularly preferred is the reaction between a matrix material precursor component having a thiol or amine group as the nucleophilic group and a matrix material precursor component having an acrylate or vinyl sulfone groups as electrophilic groups. The most preferred nucleophilic group is the thiol group. Michael type addition reactions are described in WO 00/44808 to Hubbell et al. Michael type addition reactions allow for *in situ* crosslinking of at least a first and a second matrix material precursor component under physiological conditions in a self-selective manner, even in the presence of sensitive biological materials. When one of the matrix material precursor components has a functionality of at least two, and at least one of the other matrix material precursor components has a functionality greater than two, the system will self-selectively react to form a cross-linked three dimensional matrix.

Preferably the conjugated unsaturated groups or conjugated unsaturated bonds are acrylates, vinylsulfones, methacrylates, acrylamides, methacrylamides, acrylonitriles, vinylsulfones, 2- or 4-vinylpyridinium, maleimides, or quinones.

The nucleophilic groups are preferably thiol-groups, amino-groups or hydroxylgroups. Thiol groups are substantially more reactive than unprotonated amine groups. The pH is important in this consideration: the deprotonated thiol is substantially more reactive than the protonated thiol. Therefore, the addition reactions involving a conjugated unsaturation, such as an acrylate or a quinone, with a thiol to convert two matrix material precursor components into a matrix, will often be best carried out most quickly and self-selectively at a pH of approximately 8. At pH of approximately 8, most of the thiols of interest are deprotonated (and thus more reactive) and most of the amines of interest are still protonated (and thus less reactive). When a thiol is used as the first precursor molecule, a conjugate structure that is selective in its reactivity for the thiol relative to amines is highly desirable.

Suitable first and second matrix material precursor components include proteins, peptides, polyethylene glycol (PEG), polyoxyalkylenes, poly(vinyl alcohol), poly(ethylene-co-vinyl alcohol), poly(acrylic acid), poly(ethylene-co-acrylic acid), poly(ethyloxazoline), poly(vinyl pyrrolidone), poly(ethylene-co-vinyl pyrrolidone), poly(maleic acid), poly(ethylene-co-maleic acid), poly(acrylamide), and poly(ethylene oxide)-co-poly(propylene oxide) block copolymers. A particularly preferred matrix material precursor component is based on polyethylene glycol (PEG) and is a functionalized PEG.

Polyethylene glycol provides a convenient building block. One can readily purchase or synthesize linear (meaning with two ends) or branched (meaning more than two ends) PEGs and then functionalize the PEG end groups to introduce either a strong nucleophile, such as a thiol, or a conjugated structure, such as an acrylate or a vinylsulfone. When these components are either mixed with each other or with a corresponding component in a slightly basic environment, a matrix will be formed by reaction between the first and the second precursor component, i.e. between the thiol and the acrylate or vinylsulfone. A PEG component can be reacted with a non-PEG component, and the molecular weight or hydrophilicity of either component can be controlled to manipulate the mechanical characteristics, the permeability, and the water content of the resulting matrix.

In the formation of matrices, especially matrices that are desired to degrade *in vivo,* also peptides provide a very convenient building block. It is straightforward to synthesize peptides that contain two or more cysteine residues, and this component can then readily serve as the first matrix precursor component with nucleophilic, i.e thiolgroups. For example, a peptide with two free cysteine residues will readily form a matrix when mixed with a PEG tri-vinylsulfone (a PEG having three arms with vinylsulfones at each of its arms) at physiological or slightly higher pH (e.g., 8 to 9). The gelation can also proceed well at even higher pH, but at the potential expense of selfselectivity. When the two liquid precursor components are mixed together, they react over a period of a few minutes to form an elastic gel, consisting of a network of PEG chains, bearing the nodes of the network, with the peptides as connecting links. The peptides can be selected as protease substrates, so as to make the network capable of being infiltrated and degraded by cells, as is done in a protein-based network, such as in a fibrin matrix. Preferably the sequences in the domains are substrates for enzymes that are involved in cell migration (e.g., as substrates for enzymes such as collagenase, plasmin, metalloproteinase (MMP) or elastase), although suitable domains are not limited to these sequences. One particularly useful sequence is a substrate for the enzyme plasmin. The degradation characteristics of the gels can be manipulated by changing the details of the peptide that serves as the cross-linking nodes. One may make a matrix that is degradable by collagenase, but not plasmin, or by plasmin, but not collagenase. Furthermore, it is possible to make the gel degrade faster or slower in response to such an enzyme, simply by changing the amino acid sequence so as to alter the Kₘ or k_{cat}, or both, of the enzymatic reaction. One can thus make a matrix that is biomimetic, in that it is capable of being remodeled by the normal remodeling characteristics of cells. For example, such a study shows substrate sites for the important protease plasmin. The gelation of the PEG with the peptide is self-selective.

Having protease substrates incorporated into the matrix is important when the matrix is formed from matrix precursor components comprising functional groups, like vinylsulfones, which do not lead to linkages that are hydrolytically degradable after reaction with nucleophiles, like thiols or amines. Matrices formed from the reaction of precursor components having acrylates as functional groups, preferably PEG acrylates, and precursor components having thiols as functional groups, preferably PEG thiols, do contain hydrolytically degradable bonds. Therefore, the incorporation of protease substrates allows the matrix to degrade in the body in all the cases in which the reaction of the matrix material precursor components do not lead to hydrolytically degradable bonds.

The synthetic matrices are operationally simple to form. At least two liquid matrix material precursor components are mixed; one precursor contains a precursor molecule with nucleophilic groups and the other precursor molecule contains the electrophilic groups. Physiological saline can serve as the solvent. Minimal heat is generated by reaction. Therefore, the gelation can be carried out *in vivo* or *in vitro,* in direct contact with tissue, without untoward toxicity. Thus polymers other than PEG may be used, either telechelically modified or modified on their side groups.

### PTH

The parathyroid hormone can be PTH₁₋₈₄ (native), PTH₁₋₃₈, PTH₁₋₃₄, PTH₁₋₃₁, or PTH₁₋₂₅, or any modified or allelic versions of PTH exhibiting properties, i.e. bone formation, similar to the foregoing ("PTH"). Most preferred is PTH₁₋₃₄.

### PTH Fusion Peptides

In a preferred embodiment the PTH is a PTH fusion peptide, which comprises at least two domains wherein the first domain comprises PTH and the second domain comprises a crosslinkable substrate domain crosslinkable, preferably covalently crosslinkable, to the matrix during or after its formation. The substrate domain is preferably a domain for an enzyme, preferably a substrate domain for a transglutaminase ("transglutaminase substrate domain"), more preferably for a tissue transglutaminase ("tissue transglutaminase substrate domain") and most preferred it is a substrate domain for Factor XIIIa ("Factor XIIIa substrate domain"). Transglutaminases catalyse acyltransfer reactions between the gamma-carboxamide group of protein bound glutaminyl residues and the epsilon- amino group of lysine residues, resulting in the formation of N-epsilon-(gamma-glutamyl)lysine isopeptide side chains bridges. The amino acid sequence of the PTH fusion peptide can be designed to further contain an enzymatic or hydrolytic cleavage site, thus that the PTH can be released with little or no modification to the primary structure.

Transglutaminase substrate domains and in particular, Factor XIIIa substrate domains are suitable to link the PTH fusion peptide to fibrin matrices but also to synthetic matrices during its formation. The synthetic matrix precursor components have to have pending primary amino groups in order for the transglutaminase to crosslink the Transglutaminase substrate domain of the fusion peptide to the synthetic matrix precursor components. If the PTH fusion peptide is synthesized to contain a free cysteine group, i.e. an accessible thiol group, the thiol group can react with a synthetic matrix precursor component having electrophilic groups, like acrylate groups. In a preferred embodiment the cystein group is at the N-terminal end of the PTH. The degradation site in the fusion peptide is preferably enzymatically degradable, so that the release of the PTH is controlled by cell specific processes, such as localized proteolysis.

The substrate domains covalently crosslinkable by transglutaminase and suitable for the purposes of the present invention have been described in detail including their amino acid sequences (sequence listing) in WO 03/052091.

The crosslinkable substrate domain may include *GAKDV* (SEQ ID NO: 14 in WO 03/052 091), KKKK (SEQ ID NO: 11 in WO 03/052 091), *YRGDTIGEGQQHHLGG* (SEQ ID NO: 13 in WO 03/052 091), or *NQEQVSPL* (SEQ ID NO: 12 in the sequence listing of WO 03/052 091).

The most preferred Factor XIIIa substrate domain has an amino acid sequence of *NQEQVSPL* and is herein referred to as "TG".

The PTH fusion peptide may be produced recombinantly or by chemical synthesis. The PTH 1-34 fusion peptide is preferably produced by chemical synthesis.

As described herein as a preferred embodiment the Factor XIIIa substrate domain is either directly linked to the PTH₁₋₃₄ or it can include a degradation site between the PTH (first domain) and the NQEQVSPL sequence (second domain). As such, the PTH₁₋₃₄ fusion peptide may be incorporated within fibrin or synthetic matrix during formation via a factor XIIIa substrate and afterwards released as PTH₁₋₃₄.

The degradation sites allow the PTH to be released with little or no modification to the primary peptide sequence, which may result in higher activity of the factor. In addition, it allows the release of the factor to be controlled by cell specific processes. This allows factors to be released at different rates within the same material depending on the location of cells within the material. This also reduces the amount of total PTH₁₋₃₄ needed, since its release is controlled by cellular processes.

### Degradation sites of the fusion peptide

An enzymatic or hydrolytic degradation site can be present between the first and the second domains of the fusion peptide. The degradation sites may be degradable by specific enzymatic degradation. This allows the release of the PTH to be controlled by cell specific processes, such as localized proteolysis. It allows PTH to be released at different rates depending on the location of the invading cells within the matrix. Preferably the degradation site is cleavable by an enzyme selected from the group consisting of plasmin and matrix metalloproteinase. By careful selection of Kₘ and k_{cat} of this enzymatic degradation site, degradation could be controlled to occur either before or after degradation of the fibrin matrix and/or by utilizing similar or dissimilar enzymes to degrade the matrix. These degradable sites allow the engineering of more specific release of PTH from fibrin matrices. The degradable site can be cleaved by enzymes released from cells which invaded the matrix. The degradation site allows the rate of delivery to be varied at different locations within the matrix depending on cellular activity at that location and/or within the matrix. Additional benefits include the lower total drug dose within the delivery system, and spatial regulation of release which permits a greater percentage of the drug to be released at the time of greatest cellular activity. The degradation site is abbreviated "pl" in the context of the present invention.

Proteolytically degradable sites could include substrates for collagenase, plasmin, elastase, stromelysin, or plasminogen activators. Exemplary substrates are listed below. N1-N5 denote amino acids 1-5 positions toward the amino terminus of the protein from the site were proteolysis occurs. N1'-N4' denote amino acids 1-4 positions toward the carboxy terminus of the protein from the site where proteolysis occurs.

**Table 1: Sample substrate sequences for protease.**

| Protease | N5 | N4 | N3 | N2 | N1 | N1' | N2' | N3' | N4' | disclosed in Reference |
|---|---|---|---|---|---|---|---|---|---|---|
| Plasmin | | | L | I | K | M | K | P | | 1 |
| Plasmin | | | N | F | K | S | Q | L | | 1 |
| Stromelysin | Ac | G | P | L | A | L | T | A | L | 2 |
| Stromelvsin | | Ac | P | F | E | L | R | A | NH₂ | 2 |
| Elastase | | | z- | A | A | F | A | NH₂ | | 3 |
| Collagenase | | G | P | L | G | I | A | G | P | 4 |
| t-PA | P | H | Y | G | R | S | G | G | | 5 |
| u-PA | P | G | S | G | R | S | A | S | G | 5 |

### References:

1. Takagi and Doolittle, (1975) Biochem. 14:5149-5156.
2. Smith et al., (1995). J. Biol. Chem. 270:6440-6449.
3. Besson et al., (1996) Analytical Biochemistry 237:216-223.
4. Netzel-Arnett et al., (1991) J. Biol. Chem.. 266:6747-6755.
5. Coombs et al., 1998. J. Biol. Chem. 273:4323-4328.

A preferred embodiment is the sequence YKNR (SEQID NO:19 in the sequence listing in WO 03/052 091) between the first domain and the second domain. It makes the linkage plasmin degradable.

A particular preferred PTH fusion peptide is TGplPTH ₁₋₃₄: NQEQVSPLYKNRSVSEIQLMHNLGKHLNSMERVEWLRKKLQDVHNF (SEQ ID NO: 18 in the sequence listing in WO 03/052 091)

Another preferred PTH fusion peptide is TG-PTH₁₋₃₄ which comprises the amino acids 1-34 of the native PTH as well as a TG (transglutaminase) substrate domain but no degradation site NQEQVSPLSVSEIQLMHNLGKHLNSMERVEWLRKKLQDVHNF (SEQ ID NO: 17 in the sequence listing in WO 03/052 091).

Enzymes that could be used for proteolytic degradation are numerous. Proteolytically degradable sites could include substrates for collagenase, plasmin, elastase, stromelysin, or plasminogen activators.

### Definitions

"Biological activity" as generally used herein refers to functional events mediated by a protein of interest. In some embodiments, this includes events assayed by measuring the interactions of a polypeptide with another polypeptide. It also includes assaying the effect which the protein of interest has on cell growth, differentiation, death, migration, adhesion, interactions with other proteins, enzymatic activity, protein phosphorylation or dephosphorylation, transcription, or translation.

"Spinal fusion" as used herein refers to a surgical procedure aimed at achieving bone formation and thereby fusion between two or more vertebrae.

"Calcium mineral" as generally used herein refers to naturally occurring homogenous substances which contain calcium ions. An example for a calcium mineral is hydroxyapatite (Ca₅[(OH)(PO₄)₃] which is the main component of teeth and bones.

"Cross-linking" as generally used herein means the formation of covalent or ionic linkages, preferably covalent linkages.

"Fibrin matrix" as generally used herein means the product of a process in which substantially all of the precursor components fibrinogen and thrombin crosslink in the presence of a calcium source and Factor XIIIa to form a threedimensional network. The terms matrix, gel and three-dimensional or polymeric network are used synonymously.

"Matrix" as generally used herein refers to a material intended to interface with biological systems to treat, augment, or replace any tissue or function of the tissue depending on the material either permanently or temporarily. The matrix can serve as a delivery device for PTH incorporated therein and/or as a cell-ingrowth matrix. The matrices described herein are formed from liquid precursor components which are able to form a scaffold outside or inside the body at the site of need. The terms "matrix", "gel" or biomaterials are used synonymously herein. The terms "matrix" and "gel" refer to the composition formed after the precursor components are mixed together. Thus the terms "matrix" and "gel" encompass partially or fully crosslinked polymeric networks. They may be in the form of a liquid, semi-solid, such as a paste, or a solid. Depending on the type of precursor materials, the matrix may be swollen with water but not dissolved in water, i.e. form a hydrogel which stays in the body for a certain period of time.

"PTH" as used herein includes the human sequence of PTH₁₋₈₄ and all truncated, modified and allelic versions of PTH which exhibit bone formation properties, in particular when incorporated in a fibrin matrix or other matrix. Preferred truncated versions of PTH are PTH₁₋₃₈, PTH₁₋₃₄, PTH₁₋₃₁ or PTH₁₋₂₅, Most preferred is PTH₁₋₃₄. Preferably, the PTH is human PTH, although PTH from other sources, such as bovine PTH, may be suitable.

"PTH fusion peptide" as generally used herein refers to a peptide which contains at least a first and a second domain. One domain contains a PTH, preferably PTH 1-34 and the other domain contains a substrate domain crosslinkable to a fibrin matrix or another matrix during or after its formation, e. g. upon swelling. An enzymatic or hydrolytic degradation site can also be present between the first and the second domain.

"Peptide comprising PTH" as used herein includes "PTH" and "PTH fusion peptides".

"Interbody cage" as used herein refers to a device specifically designed to be implanted in the interbody space during spinal fusion to help restore correct spine alignment and to help maintain disc space height.

"Posterior fixation" as used herein refers to a device that is intended to stabilize a spinal segment by linking two vertebra. The posterior fixation system can be implanted in the transverses or spinous processes.

### Methods of Use and application

In the preferred embodiment, the pharmaceutical product comprising PTH in a matrix, preferably in a fibrin matrix, is used in combination with an interbody cage.

The in situ gelling/formation can occur by mixing matrix material precursor solutions, preferably fibrin matrix precursor solutions, containing the PTH, with the granules (if present) and applying the mixed, preferably crosslinked composition in the interbody space. In another embodiment the mixed, preferably crosslinked composition can be applied in the interbody cage and then implanted in the prepared disc space. Depending on the indication, operational technique or preference of the surgeon the mixed material can be applied in a still liquid state or in a paste like consistence. The fibrin matrix precursor components, fibrinogen and thrombin, should be kept separate prior to mixing, to avoid premature polymerization. The same applies to precursor components of other matrices. To prevent premature contact a kit which separates the precursor solutions from each other may be used. Upon mixing under conditions that allow polymerization (crosslinking), the precursor solutions form a PTH supplemented three dimensional network. Depending on the precursor components and their concentrations, the gelling time can be tailored to the need.

Cells can also be added to the pharmaceutical composition of the present invention prior to or at the time of implantation, or even subsequent to implantation to the pharmaceutical product, i.e subsequent to crosslinking of the precursor molecules.

In a first embodiment fibrinogen is dissolved in a buffer solution (which may contain additionally aprotinin to increase stability) at physiological pH (in a range from pH 6.5 to 8.0, preferably from pH 7.0 to 7.5) and stored separately from a solution of thrombin in a calcium chloride buffer. The buffer solution for the fibrinogen can be a histidine buffer solution including additionally NaCl or TRIS buffered saline. Both solutions are stored frozen and have to be thawed prior to application.

In a preferred embodiment, a kit, which contains PTH, preferably PTH fusion protein, fibrinogen, thrombin, and a calcium source, is provided. Optionally, the kit may contain a granular material, preferably containing a calcium mineral, and a crosslinking enzyme, such as Factor XIIIa. The PTH, preferably the PTH fusion protein, may be present in either the fibrinogen or the thrombin solution. In a preferred embodiment the fibrinogen solution contains the PTH, preferably the PTH fusion peptide. Preferably the kit also contains an interbody spinal fusion cage.

The fibrinogen and thrombin solutions (first and second matrix material precursor solutions) are preferably mixed by a connector device, in which mixing occurs by squeezing the contents of one syringe into the other back and forth several times.

In a preferred embodiment both fibrinogen and thrombin are stored separately in lyophilised form. Either of the two can contain the fusion protein and the granular material. Prior to use, the tris or histidine buffer is added to the fibrinogen. The buffer may additionally contain aprotinin. The lyophilized thrombin is dissolved in the calcium chloride solution. Subsequently, the fibrinogen and the thrombin solutions are placed in separate containers e. g. vials or syringe bodies and mixed by a two way connecting device, such as a two-way syringe. Optionally, the containers/vials/syringe bodies are bipartited, thus having two chambers separated by an adjustable partition, which is perpendicular to the syringe body wall. One of the chambers contains the lyophilised fibrinogen or thrombin, while the other chamber contains an appropriate buffer solution. When the plunger is pressed down, the partition moves and releases the buffer into the fibrinogen chamber to dissolve the fibrinogen. Once both fibrinogen and thrombin are dissolved, both bipartite syringe bodies are attached to a two way connecting device and the contents are mixed by squeezing them through the injection needle attached to the connecting device. Optionally, the connecting device contains a static mixer to improve mixing of the contents.

The granular material, if present, is provided in a separate container, e. g. a syringe. According to a preferred embodiment, said granular material is wetted by injecting sterile water into said syringe. Subsequently, the above two-way syringe containing the fibrin precursor solutions and the PTH is attached to the syringe containing the wetted granular material. The entire content of the two-way syringe is transferred into the other syringe. The entire content is subsequently injected to the site of the fusion site and is moldable for several minutes. Preferred granular materials are e. g. MBCP™-Hydroxyapatite/β-tricalcium phosphate-Microgranules.

An analogous procedure is followed, if a different matrix, e. g. a synthetic matrix, is prepared. The matrix material precursor components are stored separately, and preferably mixed by a connector device as described above.

The following examples are included to demonstrate preferred embodiments of the invention. In particular, in all examples TGpl PTH₁₋₃₄ was used in a fibrin matrix, and covalently linked thereto. However, different matrix materials, e. g. synthetic matrices, and different PTH peptides, with or without TG and with or without proteolytic degradation site, may be used. The active agent, i. e. PTH, may be covalently or non-covalently linked to the matrix. It is also stressed, that the components for the kit of the present invention, the components for forming the pharmaceutical product, and the components of the pharmaceutical formulation, irrespective of whether the formulation is in an spinal fusion cage or not, are the same components as disclosed herein. The method for forming the pharmaceutical product uses the components of the pharmaceutical formulation and the kit, respectively.

### Brief description of the drawings

Fig. 1 illustrates bone mineral densities obtained with different PTH formulations in a sheep anterior cervical interbody fusion model,
Fig. 2 illustrates histomorphometry results obtained with different PTH formulations in a sheep transforaminal lumbar interbody fusion model,
Fig. 3 illustrates µCT analysis results obtained with different PTH formulations in a baboon anterior lumbar interbody fusion model, and
Fig. 4 illustrates histomorphometry results obtained with different PTH formulations in the baboon anterior lumbar interbody fusion model.

### Example 1: Sheep anterior cervical interbody fusion model

The fibrin matrix is formed from the commercially available TISSEEL VH S/D DUO® or from ARTISS (Baxter AG, CH-8604 Volketswil/ZH) giving 4mL fibrin matrix and having the composition as listed below. TISSEEL and ARTISS are produced from human derived pooled plasma and the content of active ingredients may vary from lot to lot within predefined ranges.

*Test item:* TGpl PTH₁₋₃₄ (in the experimental section TGpl PTH₁₋₃₄ and PTH or PTH₁₋₃₄ is used synonymously) in fibrin was tested alone or with granular material. PTH in fibrin was prepared using 2 separate syringes, one containing fibrinogen solution and PTH fusion peptide, and the other containing thrombin solution (syringes 1 and 2). PTH in fibrin with granular material was prepared using 3 separate syringes, a two-way syringe containing fibrin precursor solution and PTH fusion peptide (syringes 1 and 2) and two one-way syringes containing granules (syringes 3 and 4). Table 2 lists the composition of TISSEEL or ARTISS used:

**Table 2:**

| **Ingredients** | **Dose in syringe** |
|---|---|
| ***Syringe 1*** | |
| Active Component: | 0,4-10 mg |
| PTH₁₋₃₄ fusion peptide (TGplPTH₁₋₃₄) | |
| Clotting Agents | |
| Fibrinogen | 72-110 mg |
| Plasma Fibronectin | 2-9 mg |
| Other Proteins | |
| Aprotinin (Bovine) | 2250-3750 KIE |
| Factor XIII | 1-5 E² |
| Plasminogen (Human) | 0.04-0.12mg |
| Human Albumin | 10-20 mg |
| Buffer Components | |
| Niacinamide | 3-9mg |
| Histidine | 10-25mg |
| Trisodium Citrate | 4.8-7.9 mg |
| Triton WR 1339 | 0.2-0.4 mg |
| Water for Injection | To 1 mL |

| ***Syringe 2*** | |
|---|---|
| Clotting Agents | |
| Thrombin (Human) | 2.5-6.5 I.U. |
| Buffer Components | |
| Calcium Chloride | 36-44µmol/L |
| Sodium Chloride | 3.5-5.5 mg |
| Protein (human serum albumin) | 45-55 mg |
| | 25 mg |
| Water for Injection | To 1 mL |

| ***Syringe 3*** | |
|---|---|
| Water for injection | 1 mL |

| ***Syringe 4*** | |
|---|---|
| Hydroxyapatite/calcium phosphate granules (TCP granules) | 1.75-2 g |

To prepare PTH in fibrin the fibrin precursor solution of syringe 1 (fibrinogen and PTH suspended in a solution with aprotinin, a serine proteinase inhibitor which helps reduce fibrolysis to retain the integrity of the fibrin matrix) is mixed with the fibrin precursor solution of syringe 2 (thrombin in a calcium chloride solution).

To prepare PTH in fibrin with granular material first the hydroxyapatite/calciumphosphate granules in syringe 4 are wetted by injection of the water of syringe 3. Than the fibrin precursor solution of syringe 1 is mixed with the fibrin precursor solution of syringe 2 and than with the granular material.

TGplPTH₁₋₃₄ is formulated into the fibrinogen component to give a final concentration varying from of 0 mg/mL (negative control) to 1mg/mL of the fibrin matrix and during the gelation process TGplPTH₁₋₃₄ becomes crosslinked to the matrix. Fibrin precursor solutions also contain other components of fibrin matrix, such as plasma fibronectin, Factor XIII, plasminogen, and human albumin. When the precursor solutions are in equal volumes, a clotting process occurs to form fibrin, a natural extracellular matrix. The clotting process takes place over several minutes which allows for subsequent manipulation like the simultaneous injection of the mixed solutions into syringe 4, which contain the wetted granules or the application of the product.

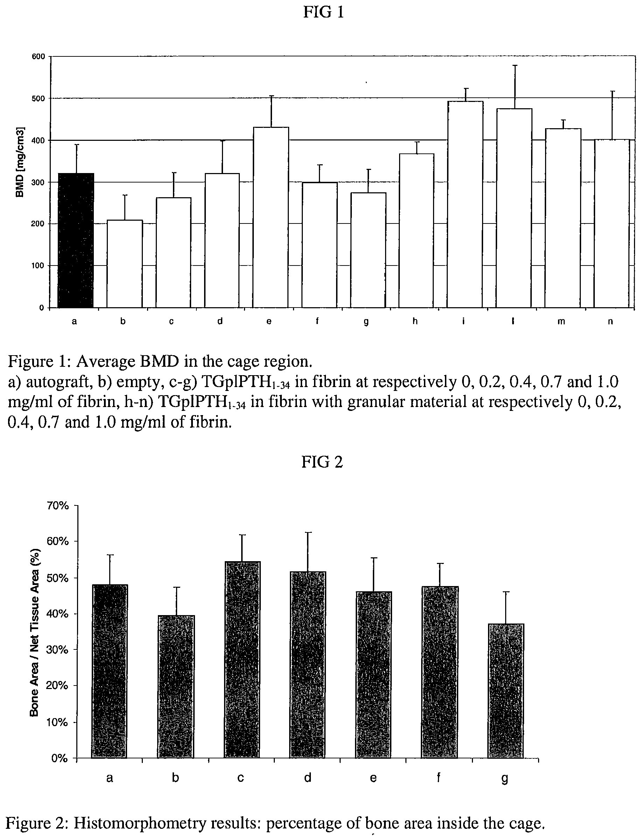

PTH in fibrin is placed in the interbody cage (0.5 ml) prior to implantation. After implantation of the cage other 3 ml of product are placed around it. *Animals*: A total of 96 Merino female sheep ranging between 51-110 kg (mean: 62.8 kg) and approximately 2 years of age were chosen as experimental animals. Twelve groups were formed (Fig. 1) with autografts as positive (a) and sham (b) as negative controls. The other groups consisted of the same fibrin matrix with (h-n) or without (c-g) granular material and with different concentrations of TGplPTH₁₋₃₄, thus the object of the present invention. Groups were followed for 12 weeks, when animals were sacrificed at the university owned slaughter house. All animal experiments were conducted according to the German laws of animal protection and welfare and were authorized by the local Ethical Committee and Veterinary Authorities .

Before surgery and for at least 10 days thereafter the animal where housed for in inside stalls. Here the health status of the animals was controlled daily for any signs of inflammation or toxicity as represented by pain, infection of the wound, local swellings and/or changed general behavior e.g. food intake, excretion of urine and faeces, excitement, apathy etc. After their return to pasture, their health status was controlled daily by the animal keepers of the LGF-Versuchsstation Nutztierwissenschaften, Lentzealle 75, 14195 Berlin. With any deterioration in health, sheep were returned to the animal facility of the Virchow-Klinikum, Charité-Universitätsmedizin Berlin where they were examined by the veterinarians.

*Surgery*: The cervical spine was accessed ventrally. Using an image amplifier, the intervertebral space C3/C4 was identified. After dissection of the ligamentum longitudialis anterior, a discectomy was performed and the adjacent endplates decorticated. Subsequently, the intervertebral space was expanded using a cylindrical bur and an interbody fusion cage (SynCage-C, curved, blue, size 7.0, REF 495.302, SYNTHES®, Synthes GmbH, Eimattstrasse 3, 4436 Oberdorf, Switzerland) pre-filled with the biomaterials, or autograft or left empty (sham control) inserted into the prepared intervertebral space. Additional test material was then placed around the cage. The surgical access was closed by four suturing.

For the positive autograft control group, autogenic bone was harvested from the dorsal part of the left iliac crest through a local incision. The iliac crest was opened using a hammer and chisel. Two cuts at a distance of 3 cm were made and connected on one side, so that this part of the iliac crest could be flapped over like a cover. The cancellous bone was removed using a sharp spoon. The cover was then replaced and fixed into position by a filament (Vircryl®Plus 1, J&J, Belgium). To close the surgical access, the subcutis was sutured continuously using a resorbable filament (Vircryl®Plus 3-0, J&J, Belgium). Finally, the skin was closed with a non-resorbable filament (Prolene®, 3-0, Ethicon GmbH, Norderstedt, Germany).

*Evaluative procedures*: Animals were sacrificed after 12 weeks and the spine segment comprising the level C3/C4 was extracted for final analysis. Four different techniques were employed to evaluate the outcome on spinal fusion and included radiography, non-destructive biomechanical testing, computed-tomography (CT), and histological examinations

*Radiological evaluation*: Radiographs were taken before and directly after surgery, as well as 8 and 12 weeks thereafter (radiography unit: Mobilett Plus, Siemens AG, Fuji CR 24x30, Fuji). Radiographic evaluation was performed by two independent reviewers with respect to three different parameters: disc space height, intervertebral angle and radiographic fusion. All measurements were repeated three times and the final results reviewed by the principal investigator.

Regarding disc space height, anterior, central and posterior intervertebral disc space height of the motion segment C3/C4 was measured on lateral radiographic scans. Subsequently, average intervertebral disc space height was calculated from anterior, central and posterior measurements.

The intervertebral angle was also measured on lateral radiographic scans.

A semi-quantitative score system was developed to evaluate the radiographs. High scores were favourable for bone healing.

*Computed tomography*: After sacrifice, peripheral quantitative computed tomographic (pQCT) scans were performed (Siemens Somatom Plus, Siemens, Erlangen). Axial cuts with a 1 mm slice thickness were made parallel to the intervertebral disc space.

Bone mineral densities (BMD) were determined as described by Kandziora and Pflugmacher (Kandziora *et al.,* 2001 and 2004, Pflugmacher *et al.,* 2005). BMD measurements were calibrated with a 6-point bone mineral density phantom.

Four distinct and separate regions of interest were identified inside the cage and an individual measurement of BMD was made for each region. The chosen regions were identical between each sample to ensure comparability of the results. For defining the regions, the holes present in the spinal fusion cages were taken as landmarks. Additionally, four regions in the vertebral bodies C3 and C4 were measured.

Measurements were performed using specific scanner software (Sienet Magic View VA 30A, Siemens, Inc., Erlangen, Germany). All parameters were evaluated by three independent reviewers.

*Non destructive biomechanical testing:* After the animals were sacrificed, the biomechanical properties of the treated segments were evaluated by non-destructive mechanical testing. Pure bending moments were applied to the motion segment C3/C4 using a system of cables and pulleys to induce flexion and extension, left and right lateral bending and left and right axial rotation. Tension was applied to the cables with a uniaxial testing machine (Zwick 1456, Zwick GmbH, Ulm, Germany) (Kandziora *et al.,* 2001).

Three-dimensional displacement of each motion segment was measured using an optical measurements system (Qualysis, Sävebalden, Sweden). Two triangular markers with three diodes (Qualysis, Sävebalden, Sweden) were attached to the C3 and C4 vertebral bodies. Marker positions were detected with two cameras and recorded using a computer-controlled system to study micromotions (PC-Reflex; Qualysis Inc., Sävebalden, Sweden). Angular displacement of C3 in relation to C4 was calculated by using custom designed software.

Vertebral bodies were embedded in a two component plastic (Beracryl powder, Beracryl monomer, Bauer Handels GmbH Waberg, Adetswil, Switzerland) to ensure proper fixation and that the lower extremity of the segment was attached rigidly to the base of the testing apparatus. The weight of the apical fixation device resulted in an axial preload of 25 N, corresponding approximately to the weight of a sheep head. The samples were kept wet during the whole testing procedure. Moments were applied in increments of 1 Nm per second up to a maximum of 6 Nm. Specimens were first preconditioned with three cycles. Data was then acquired during the application of the fourth cycle. The range of motion (ROM), the neutral and elastic zone, as well as the stiffness values were determined from the corresponding torque-angular displacement curves.

*Histology.* After biomechanical testing, the vertebral C3/4 motion segments were subjected to histological analysis of non-decalcified, parasagittal sections using different stains to assess the effects of the biomaterials. Histomorphological and histomorphometric analysis, as well as a determination of the histological fusion was performed.

Firstly, the segments were fixed for one day in 10% normal buffered formaldehyde. Then the segments were cut sagitally and parasagitally in 8 slices of 4 mm and subsequently fixed for a further 7 days in 10% normal buffered formaldehyde. Afterwards, the residual parts of the cages were removed and the slices were dehydrated in ascending concentrations of ethanol. After embedding, either in paraffin or in methyl methacrylate, longitudinal sections of 6 µm were cut in the sagittal plane (Leica SM 2500S Microtome) and the following coloration techniques used:
1. alpha smooth muscle
2. TRAP
3. Safranin orange/von Kossa
4. Movat pentachrom
5. Methyl green/van Gieson
6. Toluidine blue
7. Masson Goldner

The histological fusion score within the cage was evaluated based on one central slide by two independent reviewers on the Safranin orange/von Kossa stained slices using a three scale scoring system.

*Histomorphometry* Sections stained by Safranin orange/von Kossa, Methyl green/van Gieson and Movat pentachrom were used for histomorphometry. Parameters were measured using a Leica DM-RB microscope and an image analysis system (Zeiss KS 400, Zeiss GmbH, Germany).

First, region(s) of interest (ROI) were defined. As a first region, an overall ROI was defined by the width of the original intervertebral disc and a height of 35 mm. Inside this ROI, 5 different sub regions were defined. The cage ROI comprised a rectangle of 7 mm in height and 12 mm in width at the location of the cage. Adjacent to the cage ROI, a dorsal and ventral ROI were defined ranging from the cage to the dorsal or ventral end of the vertebra, respectively. Furthermore, a cranial and caudal ROI were defined comprising 5 mm of the cranial (C3) or the caudal (C4) vertebra adjacent to the cage, respectively (Figure 6).

The following structural indices were calculated in the ROI:
1. bone volume/total volume
2. cartilage volume/total volume
3. volume of connective tissue/total volume
4. volume of granules/total volume (only for groups treated with the biomaterial containing granular material)

Bone and connective tissue were determined from Safranin orange/von Kossa stains, cartilage from Movat pentachrom stains and granules from Methyl green/van Gieson stains.

Histomorphology:Sections stained by alpha smooth muscle, TRAP, Safranin orange/von Kossa, Masson Goldner, Movat pentachrom and for the Bone Graft Substitute groups additionally Methyl green/van Giesson were used for histomorphology.

Histomorphological analysis was performed in the five ROI to determine ventral fusion, presence of woven bone, hyaline cartilage, potential foreign body reaction, vascularization, osteolysis and the occurrence of osteoclasts and osteoblasts. Two separate reviewers performed the evaluation of the different groups: one evaluated the autograft and the biomaterial without granules; the other evaluated the sham and the biomaterial with granules.

The results of these experiments are shown in Fig. 1:
A low level of bone formation was observed in empty cages, while significant bone formation was observed with autograft.

Treatment with the biomaterial without granules showed clear bone formation within the spinal fusion cage and thus, promotion of spinal fusion. Dependent on the analysis technique used, a dose dependency with best results from TGplPTH1-34 at 0.2 and 0.4 mg/mL fibrin was found. On measuring BMD, radiographic fusion, maintenance of disc space height and intervertebral angle, similar or better results than autograft were shown.

The biomaterial with granules showed similar results in the radiological parameters than autograft; however, granules were still present at sacrifice in animals, which may have led to an overestimation of the radiographic fusion score and BMD for these groups.

Histomorphometric analysis demonstrated that all the groups treated with the biomaterial without granules showed an increase percentage of bone compared with empty. Moreover, the biomaterial containing 0.2 mg of TGplPTH1-34 /mL fibrin presented comparable results to autograft in the percentage of bone, cartilage and connective tissue.

The biomaterial with granules presented low percentage of bone in comparison to autograft, best results were obtained with a concentration of 0.2 mg of TGplPTH1-34 /mL fibrin.

Whereas bone formation within the spinal fusion cage was observed, the biomaterials showed poorer stabilization than autograft, as assessed by biomechanical testing. This may be due to the early time point that was chosen for the evaluation of spinal fusion, being too short to allow significant mechanical stabilization.

Histomorphology analysis showed inflammatory cells and increased amounts of blood vessels in the proximity of the titanium cages in some animals left empty or treated with autograft, or with the biomaterials, but no trend related to the concentrations of TGplPTH1-34 was observed. Also, in the groups treated with the biomaterial containing granules areas of mild inflammation where observed around the granules outside the cage, but this was again unrelated to the TGplPTH1-34 concentration. Overall the product appears very biocompatible.

The improved results in the group treated with TGplPTH₁₋₃₄ compared to the control groups demonstrated that the presence of clinically relevant doses of TGplPTH₁₋₃₄ in the biomaterials of the present invention can lead to a strong improvement in healing. The faster healing, and improved bone formation are relevant characteristics to justify application of the biomaterial of the present invention in spinal fusion procedures. Furthermore, when the results of the radiological evaluation, BMD and histological fusion are evaluated, it can be seen that doses between 0.2 mg/mL to 0.7 mg/mL TGplPTH₁₋₃₄ fibrin matrix is the most preferred concentration range.

### Example2: Sheep transforaminal lumbar interbody fusion model

As in example 1, PTH (i. e. TGpl PTH₁₋₃₄) was tested alone and with granular material, respectively. The same materials were used as in example 1, and the preparation of the product followed the same protocol as for example 1, but in this study the concentrations of TGplPTH₁₋₃₄ ranged from 0.2 mg/mL to 0.7 mg/mL of the fibrin matrix. The treatment was placed only inside the interbody cage. No additional test material was placed around the case, as in example 1.

*Animals:* A total of 28 Crossbred Suffolk Sheep (Castrated Weathers) of average weigh of 57 kg and from 3 to 6 years of age were chosen as experimental animals. Seven groups were formed. Autograft was used as positive control and the other groups consisted of the same fibrin matrix with or without granular material and with different concentrations of TGplPTH₁₋₃₄, thus the biomaterials of the present invention. Groups were followed for 16 weeks, when animals were sacrificed. Animal care and use was conducted in accordance with federal regulations as outlined in the Animal Welfare Act. Surgery, perioperative care, housing, sanitation practices, husbandry, and veterinary care followed the recommendations of the NIH Guide for the Care and Use of Laboratory Animals (HHS, NIH Pub. No. 85-23, 1985). Animal care personnel were qualified, through training and experience, to perform required duties.

All animals received physical examinations by a veterinarian within two weeks prior to surgery to ensure normal health status. Any animals determined pre-operatively or during surgery to have an osseous abnormality problem (e.g. tumor, osteomyelitis) or other disqualifying factor were excluded from the study. Animals were provided a 6'x 10' pen pre-operatively and for the entire post-operative period. An isolation pen was available for special procedures and treatments, if necessary.

Prior to surgery, all animals were under food restriction (NPO) for forty-eight hours and housed in the isolation pen care facility. All animals were examined daily by the Study Director for the first ten days post-operatively with observations of mortality, wound site healing, ambulatory function, clinical signs of ill health, and behavioral (social) changes. After ten days post-operatively, these observations were performed and recorded by the animal care personnel technicians. Particular attention was given to the surgical site with an emphasis on wound healing and signs of infection, if any. Animals judged to be abnormal by the facility staff were referred to and examined by the Study Director and a veterinarian daily. Observations and serious adverse events were recorded on the case report forms and commented on with regard to diagnosis, severity, treatment, and outcome.

*Surgery*: Following intravenous administration of anesthetic medications and induction of general anesthesia, the anterolateral lumbar region and iliac crest was aseptically prepared. Following lateral recumbency positioning of the animal, surgical exposure consisted of a ten to fifteen centimeter incision beginning at the iliac crest and extending along the palpable borders of the lumbar transverse processes along the anatomic right. Detachment of the external abdominal oblique from the transverse processes was performed, followed by soft tissue dissection to permit retroperitoneal exposure of the psoas muscle. Using a periosteal elevator and electrocautery, the anterolateral aspects of the L2-L3 and L4-L5 vertebral bodies and intervertebral discs were exposed. The ventral nerve roots crossing over the disc surface were transected, followed by diskectomy and burr removal of the vertebral endplates to a dimension of approximately 24mm depth x 11mm wide x 11mm height at both operative levels. Caspar pin segmental distraction was performed at each operative level to assist in endplate removal and interspace preparation. Once prepared, the Tetris® PEEK cage (Signus Medical, LLC) was filled with 1.6 mL according to the treatment randomization schedule and securely implanted within the disc space. 100% morselized cancellous iliac autograft served as operative control. Each animal received the same treatment at both operative levels to avoid cross contamination of different dosages. Treatments were performed according to a pre-defined randomization table, strictly according to the manufacturer's recommendations for device implantation. For wound closure, the muscle, subcutaneous layer, and skin were approximated with running and interrupted 1-0 Vicryl sutures."

Under the same anesthesia, the animal was re-positioned prone, aseptically prepared and draped in sterile fashion. An initial skin incision was made in the dorsal mid-line of the low back centered over the L2-S1 levels. Blunt dissection using a Cobb elevator and electrocautery, when necessary, was performed in the sagittal plane along the neural arch - permitting exposure of the L2-L3 and L4-L5 facets and transverse processes. After fluoroscopic verification of the previously implanted cage, the two operative levels (L2-L3 and L4-L5) were instrumented using transpedicular screw and rod fixation (S4® Spinal System - Aesculap Spine USA, Inc.). For wound closures, the muscle, subcutaneous layer, and skin were approximated with running and interrupted 1-0 Vicryl sutures and the skin closed with staples. Data on anesthesia type and duration, surgery duration, estimated blood loss, treatment assignment, complications, antibiotics and analgesics were recorded at the time of the surgical procedure.

*Evaluative procedures*: Animals were humanely sacrificed after 16 weeks. The spinal column of the animal was carefully removed and immediately placed in double wrapped plastic specimen bags and frozen at -25 degrees C° for subsequent radiographic, biomechanical and histologic evaluation of the operative motion segments.

*Radiological evaluation*: Anteroposterior and lateral fluoroscopic images of the lumbar region were obtained intra- and post-operatively to verify implant placement. Plain films were obtained post-operatively and at animal sacrifice to evaluate implant location and endplate radiolucencies. For each operative segment, computed tomography images were obtained between the cephalad and caudal pedicle levels of the arthrodesed segments in one-millimeter-thick slice intervals (Dedicated Imaging, Inc., Baltimore, Maryland). CT images were used to qualitatively assess the success of interbody fusion and location of the graft materials within the treatment level, incidence of spinal stenosis and / or ectopic calcification anterior or lateral to the implanted cage. Arthrodesis sites were graded as fusion, partial or non union by four individuals blinded to the treatment group. Fusion was defined as yes if at least two sagittal slices on the CT images demonstrated evidence of contiguous bone from endplate to endplate without signs of radiolucencies, while partial union was based on one sagittal image containing contiguous bone. Anteroposterior and lateral plain films of the lumbar region were obtained intra- and post-operatively to verify implant placement.

*Non destructive biomechanical testing*: In preparation for biomechanical testing, the lumbar motion segments were thawed to room temperature and cleaned of all residual musculature with care taken to preserve all ligamentous attachments and operative motion site integrity. The cephalad and caudal ends of each specimen were secured in rectangular containers using eight compression screws. A total of three Plexiglas motion detection markers were placed on the specimen: Marker 1 - superior element, Marker 2 - inferior element and Marker 3 - base. Each marker was equipped with three non-co-linear light emitting diodes designed for detection by an optoelectronic motion measurement system (3020 OptoTrak System). To determine the multidirectional flexibility properties, six pure, unconstrained moments: flexion and extension (±4Nm X-axis), left and right lateral bending (±4Nm Z-axis) and left and right torsion (±4Nm Y-axis) were applied to the superior end of the vertically oriented specimen while the caudal portion of the specimen remained fixed to a testing platform. A maximum applied moment of ± 4Nm was used for each loading mode and applied at a ramp rate of three degrees/second using a six degree of freedom spine simulator (6DOF-SS). A total of three load / unload cycles were performed for each motion, with data analysis based on the final cycle. For the six main motions - corresponding to the moments applied - the operative level vertebral rotations (degrees) were quantified in terms of peak range of motion (ROM). To prevent desiccation during assessment, specimens were moistened with 0.9% NaCl sterile irrigation solution.

*Destructive biomechanical testing:* A destructive mechanical test was undertaken to quantify the structural material properties of the tissue within the PEEK cage. One L4-L5 operative motion segment was assessed for each treatment group. The operative motion segments were transversely sectioned directly along the superior and inferior edges of the PEEK cage using a Beuhler Isomet Cutting system and diamond wafering blade. Two parallel surfaces were obtained using this technique. The explanted cage was then mounted on the testing platform of an MTS 858 Bionix Test system. Indentation compression tests were performed using a 3mm diameter punch indenter at a displacement rate of 0.2mm per second to a depth of 4mm. Three tests were performed on each side of the superior and inferior surfaces with calculation of peak failure load (Newtons) for each test site from the load-displacement curves (n=6 indentation sites per cage). A total of eight cages were tested (one per group) and compared to an intact nonoperative control. The operative specimens were subsequently processed using undecalcified technique to characterize the histological tissues present at each test site and correlate these findings with the observed mechanical properties. These mechanical testing methodologies have been previously reported: (Grant JP et al: Spine 15;26-8,:28(8)896, 2001) and (Oxland TR et al: Spine 15;28(8)771-777, 2003)

*Histopathologic and histomorphometric evaluation:* Following biomechanical analysis, the operative lumbar motion segments not included in the destructive mechanical testing were sagittally sectioned along the geometric centerline of the implanted device using a Beuhler Isomet saw. The tested cage samples were also processed using undecalcified technique but sectioned coronally. Histologic preparation of all samples included dehydration in 100% ethanol, staining using the Villanueva Osteochrome Bone Stain, undecalcified solution processing and embedding in polymethyl-methacrylate (PMMA). Using the EXAKT Microgrinding Device, the embedded specimens were cut to 300 to 600 um in thickness, ground and polished to 130 um. Microradiographs were obtained of the slide-mounted specimens using Faxitron radiography. The slides were placed twelve inches from the beam and exposed for two minutes, using a technique of 34 kVp and 2mA while in direct contact with the single emulsion high-resolution graphics arts film. The high-resolution microradiographs and light microradiographs were used for fusion assessment by two reviewers blinded to the treatment groups. Fusion was defined as contiguous bone from endplate to endplate without evidence of interruptive radiolucencies within the cage. Partial union was evidenced by one contiguous strand of bone bridging the endplates, while non-union was a continuous radiolucent gap spanning the cage. Histomorphometric quantification of seven different parameters within the confines of the PEEK Cage included the following: Region of Interest (ROI) (mm²), PEEK Implant Area (mm²), Granules Area (mm²), Bone Area (mm²), Marrow Area (mm²), Net Tissue Area (mm²) and Trabecular Bone / Net Tissue Area (%). The net tissue area represents the region of interest minus the PEEK area. The PEEK cage samples included in the destructive indentation testing were evaluated using plain light microscopy to characterize tissues present.

For all specimens, the spinal cord (n=2 per specimen) and local tissue overlying the implant sites (n=2 per specimen) were resected, sectioned and prepared by a veterinarian pathologist. The specimens were fixed in a 10% formalin solution and underwent routine paraffin processing and slide preparation by the University of Maryland Biotechnology Institute (UMBI) Histology Laboratory (UMBI, Baltimore, MD). Using thin-sectioning microtomy, the paraffin embedded sections were cut (3-5µm in thickness), slide mounted and stained using standard Hematoxylin and Eosin (H&E) and special Macrophage Stain (HAM-56). Pathological assessment for all tissues included comments on tissue architecture, presence of wear debris, incidence of mineralization in local tissue / spinal cord histology, osteolysis as well as any signs of foreign body giant cell / granulomas inflammatory reactions, degenerative changes or autolysis.

The results of these experiments are shown in Fig. 2:
No toxicity was observed during the treatment period with any of the TGplPTH₁₋₃₄ doses or formulations used.
Histopathologic review of all treatment groups combined indicates no evidence of foreign body/inflammatory reaction or significant pathological changes. Many treatments contain interpositional pockets of cartilaginous/collagenous tissue, which is actively remodeling. In almost all cases, the trabecular bone within the PEEK cages is densely woven and sclerotic in most regions, containing normal osteocyte distribution and osteoid seam widths. Overall, there are no signs of osseous pathology due to any of the formulation or dosages of TGplPTH₁₋₃₄ utilized in this investigation. All histological specimens can be characterized as unremarkable and undergoing a normal healing process, without evidence of giant cell reaction/inflammatory response or other significant histopathological changes.

Plain film radiographic analysis demonstrated no evidence of implant migration. Sagittal and coronal CT images showed successful fusions in the autograft and in the treatment with the biomaterials. For the biomaterial without granules best results were obtained at a concentration of TGplPTH1-34 comprised between 0.4 and 0.7 mg/ml, while for the biomaterial with granules best results were obtained at a concentration of TGplPTH of 0.2 mg/ml. Histological fusion scoring confirmed the radiographic outcome. In the histomorphometric quantification, the percentage of bone area in the cage was highest for the 0.4 mg/mL TGplPTH₁₋₃₄ in the biomaterial without granules followed by the 0.7 mg/mL group and the autograft group.
Biomechanical testing was conducted to determine the multi-directional flexibility properties of the lumbar segments, where better stability is indicated by a lower range of motion. Results showed that TGplPTH₁₋₃₄ at 0.4 mg/mL in the biomaterial without granules and all TGplPTH₁₋₃₄ concentrations in the biomaterial with granules provided mechanical stability comparable to autograft. In general, the granules confer structural support and may contribute to mechanical stabilization.

The results obtained in the group treated with selected concentration of TGplPTH₁₋₃₄ in the biomaterials are comparable or better than the positive control groups demonstrating that the presence of clinically relevant doses of TGplPTH₁₋₃₄ in the biomaterials of the present invention can lead to an effective bone graft substitute for spinal fusion. Furthermore, when the results of the radiological, biomechanical and histological evaluation are considered, it can be seen that doses between 0.4 mg/mL to 0.7 mg/mL TGplPTH₁₋₃₄ fibrin matrix is the most preferred concentration range in the biomaterial without granules and doses between 0.2 mg/mL to 0.7 mg/mL TGplPTH₁₋₃₄ fibrin matrix is the most preferred concentration range in the biomaterial with granules.

### Example 3: Baboon anterior lumbar interbody fusion model

The same materials were used as in the previous examples (except for granular material, which was not present in the TGplPTH₁₋₃₄-fibrin composition of this example).

*Test item:* TGplPTH₁₋₃₄ is formulated into the fibrinogen component to give a final concentration of 0.4 mg/mL of the fibrin matrix.

*Animals:* A total of 3 Olive or Yellow Baboon of approximate weight of 30 kg) and 8 - 11 years of age were chosen as experimental animals. The baboons were subjected to a L4/L5 and L6/L7 discectomy and fusion using a radiolucent vertebral cage. In each animal one level contained a total volume of 0.7 mL of autologous bone graft inside the cage (positive control), whereas the other level contained same volume of 0.4 mg of TGplPTH₁₋₃₄ per mL of fibrin. The two interventional segmental levels were blockrandomized to the autograft control and the treatment group. The animals were followed for 6 months, when animals were sacrificed. All animal experiments were conducted at the Southwest Foundation for Biomedical Research (SFBR), 7620 NW Loop 410, San Antonio, TX 78227, USA according to the laws of animal protection and welfare ("Animal Welfare Act" and "Guide for the Care and Use of Laboratory Animals"). The experiment was authorized by the Institutional Animal Care and Use Committee (IACUC).
Before and after surgery, animals were checked regularly for their general health status.

*Surgery*: The animals were placed in a supine position. Surgical exposure consisted of a 10-15 cm incision in the left lateral abdominal wall, followed by soft tissue dissection to allow retroperitoneal exposure of the anterior lumbar spine. Blunt dissection using a Cobb elevator and electrocautery was performed as needed, to expose the anterior aspects of the L4/L5 and the L6/L7 vertebral bodies with interposed intervertebral discs. Intra-operative radiography was used to verify the operative levels. The aorta and vena cava overlying the anterior lumbar spine were gently retracted and two non-contiguous disc sites (L4/L5 and L6/L7) were prepared in the lumbar spine, for insertion of the implant spacers. For this purpose, a rectangular window approximately 15 millimeter (mm) wide, centered and extending from the caudal to the cranial endplate rim was cut into the discs. The disc material was gradually removed using small rangeurs and curettes and until the endplates were bleeding from the whole cross-sectional area. A PEEK cage, properly sized to find a tight fit, was filled with either autologous bone or TGplPTH₁₋₃₄ in fibrin, before insertion into the prepared intervertebral disc space. For the autograft treatment, cortico-cancellous bone graft chips were harvested using a curved chisel and a hammer along the inside of the left anterior ileum wing. Care was taken not to violate the outer cortical shell of the wing. The chips were then further cut with a bone rongeur into smaller pieces that were used to densely pack the inside of the cage. For wound closure, the muscles and fascia were approximated using 1-0 Vicryl and the skin closed with 2-0 Vicryl sutures. The implant position was documented directly after surgery by radiographs in antero-posterior and lateral view.

*Evaluative procedures*: Blood and radiological analysis were performed every 4 weeks until sacrifice. During the follow up time various fluorescent compound were administered in order to allow the monitoring of calcium apposition over time. Animals were sacrificed after 24 weeks and the vertebral segments L4/L5 and L6/L7 were harvested for final analysis. Three different techniques were employed to evaluate the surgical results. These included radiography/microradiography, micro computed tomography (µCT) and histological examinations.

*Radiological evaluation*: Radiographs were taken in antero-posterior and lateral projections before and directly after surgery, as well as every four weeks thereafter up to 24 weeks following surgery. Films were used to qualitatively assess the evolution and success of interbody fusion through the study, as well as to identify cage migration and reduction of the intervertebral space. Radiographic evaluation was performed by two independent reviewers blinded to the treatment group following a semi-quantitative scoring system from 0 to 3 for the fusion and from 0 to 2 for cage displacement and disruption of vertebral space.

*Computed tomography*: µCT analysis was performed with respect to the bone present in the spinal fusion cage. An algorithm was used to create a mask representing the inner volume of the cage. Firstly, a region of interest equal to the inner shape of the spinal fusion cage was defined. Within this region of interest, bone volume was analyzed and then Bone Volume divided by Total Volume (BV/TV) was calculated.

*Histology:* Histological analysis was performed qualitatively and semi quantitatively. For each level, three thick histological sections were prepared for the midsagittal plane, as well as for both para-sagittal planes with an offset of about 4 mm. After flattening by weights for two days, microradiographs were taken (Faxitron), then ground to a thickness of 30-40 micrometer (µm) and stained with Toluidine blue. Qualitative analysis of histological sections were conducted focusing on the fusion within the cage, cage displacement, disc material / cartilage, and callus formation (scoring 0 to 3) From each level two histological thin sections were prepared to semi quantitative score the presence of osteoblast, osteoclast, foreign body cells, lymphocytes, macrophages and plasma cells. Histological thin sections were prepared and stained with Toluidine blue and Von Kossa / McNeal Tetrachrome counterstain.

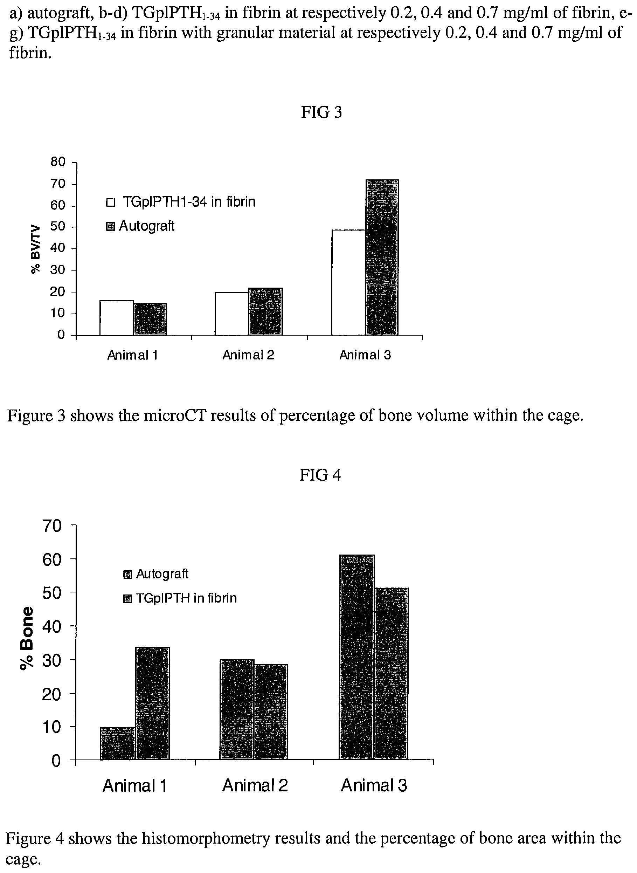

*Histomorphometry:* Quantitative histology is performed blinded to the experimental groups. First, the pictures are coloured for different features (e.g. fibrous tissue, old bone, new bone etc.) using AdobePhotoshop®. The following histomorphometry is performed by a standardized and automated procedure using a Leica microscope to quantify. The percentages of bone, cartilage and connective tissue were calculated.
The results of these experiments are shown in Fig. 3 and Fig. 4.

No safety issue were seen with the use of the material and in particular no hypercalcemia was seen in the blood analysis.
Radiographic, histological and µCT analysis demonstrated a reduction of the intervertebral space in most levels, which may be secondary to the surgical procedure. An anterior migration of the Poly-Ether-Ether-Ketone (PEEK) cage was also observed in some levels, which could have created the loss of some material.

Radiographic fusion showed complete bridging or clear bridging with a small intervertebral gap in all animals in the treatment groups TGplPTH₁₋₃₄ in fibrin, with a similar fusion trend than autograft internal control.

Histological fusion scoring was performed based on the percentage of bony bridging within the cage. In general a low level of fusion was observed with only bony bridging in one level treated with autograft. Looking at the bone deposition within the cage overtime, autograft and TGplPTH₁₋₃₄ in fibrin showed bone formation at 8 and 13 weeks, but not afterwards.
µCT analysis (Fig. 3) and histomorphometry (Fig. 4) showed consistent results. The level of bone formation was variable among animals, but comparable between the treated and the control level.

Histomorphological analysis showed no major differences in terms of cell presence (osteoblast, osteoclast, foreign body cells, lymphocytes, macrophages and plasma cells) between control autograft and treatment group.
This study showed that TGplPTH₁₋₃₄ in fibrin is able to promote spinal fusion in a nonhuman primate model with efficacy and timing comparable to ileac crest autograft.

## Claims

1. A pharmaceutical formulation for use in a spinal fusion method, comprising:
a composition for forming a matrix, comprising at least a first matrix material precursor component and a second matrix material precursor component, cap - able of forming the matrix by crosslinking of the precursor components under ap - propriate conditions,
a bioactive factor, which is biologically active to stimulate bone formation between two vertebrae, and for effecting or supporting spinal fusion, the bioactive factor being suitable to be releasably incorporated in the matrix upon crosslinking of the matrix material precursor components
wherein the bioactive factor is PTH or a peptide comprising PTH, and further
wherein PTH or the peptide comprising PTH is the sole added bioactive factor which is effective to stimulate bone formation between two vertebrae and for ef - fecting or supporting spinal fusion.

2. The pharmaceutical formulation of claim 1 wherein the formulation for forming a matrix and the bioactive factor are provided as liquid components.

3. The pharmaceutical formulation of claim 1 or 2, further comprising a granular material, preferably a biodegradable ceramic compound.

4. The pharmaceutical formulation of any one of claims 1 to 3, wherein the pep - tide comprising PTH is a PTH fusion peptide comprising at least two domains wherein the first domain comprises PTH and the second domain comprises a crosslinkable substrate domain.

5. The pharmaceutical formulation of claim 4, wherein the second domain of the PTH fusion peptide comprises a transglutaminase substrate domain, preferably a Factor Xllla substrate domain.

6. The pharmaceutical formulation of claim 4 or 5, wherein the PTH fusion peptide further comprises an enzymatic or hydrolytic degradation site between the first and the second domains.

7. The pharmaceutical formulation of claim 6, wherein the degradation site is a substrate for plasmin..

8. The pharmaceutical formulation of any one of claims 1 to 7, wherein the peptide comprising PTH is TGpl-PTH₁₋₃₄.

9. The pharmaceutical formulation of any one of claims 1 to 7, wherein the matrix is fibrin, and the composition for forming the matrix comprises fibrinogen, thrombin and a calcium source.

10. The pharmaceutical formulation of claim 9, wherein the composition for forming the fibrin matrix comprises (i) fibrinogen and thrombin as first and second matrix material precursor components, and optionally Factor Xllla, and (ii) PTH in a range of between 0.01 to 1 mg PTH/mL fibrin matrix, preferably between 0.2 to 0.7 mg PTH/mL fibrin matrix, most preferably 0.4 mg PTH/mL fibrin matrix.

11. The pharmaceutical formulation of any one of claims 1 to 8, wherein the matrix is a synthetic matrix or a mixed synthetic/natural matrix, and the composition for forming the matrix comprises a matrix material precursor component having strong nucleophilic groups or bonds, and a matrix material precursor component having strong electrophilic groups or bonds.

12. The pharmaceutical formulation of any one of claims 1 to 11, wherein the treatment is interbody spinal fusion.

13. A kit for use in a spinal fusion treatment method comprising a pharmaceutical formulation as claimed in any one of claims 1 to 12, wherein at least one of the matrix material precursor components capable of forming the matrix by crosslinking of the precursor components under appropriate conditions is provided separately from the remaining matrix material precursor components.

14. The kit of claim 13, wherein the first matrix material precursor component is provided as a first precursor solution in a first container, the second matrix material precursor component is provided as a second precursor solution in a second container, and PTH or the peptide comprising PTH is provided in the first or the second precursor solution.

15. The kit of claim 13 or 14, wherein the kit contains a spinal fusion device, preferably an interbody spinal fusion cage.

16. A pharmaceutical product for use in a spinal fusion method comprising a matrix and PTH or a PTH containing peptide releasably incorporated in the matrix, wherein the pharmaceutical product is obtainable by crosslinking of the pharmaceutical formulation claimed in any one of claims 1 to 12, or obtainable by using the kit as claimed in any one of claims 13 to 15.

17. An interbody spinal fusion cage containing a pharmaceutical formulation as claimed in any one of claims 1 to 12, or containing a pharmaceutical product as claimed in claim 16.

18. A method for forming a pharmaceutical product as claimed in claim 16, comprising
providing a first matrix material precursor component,
providing a second matrix material precursor component,
providing PTH or a peptide comprising PTH, and
mixing the first and the second matrix material precursor components and PTH or the peptide comprising PTH, preferably under physiological conditions, to form a matrix material by crosslinking of the first and second matrix material precursor components, the matrix material having PTH or the peptide comprising PTH releasably incorporated therein.

19. The method of claim 18, further comprising
providing a granular material,
wetting the granular material, and
mixing the wetted granular material and the mixture of the first and the second matrix material precursor components and PTH or the peptide comprising PTH.

## Patentansprüche

1. Pharmazeutische Formulierung zur Verwendung in einem Wirbelsäulenfusionsverfahren, aufweisend:
eine Zusammensetzung zum Bilden einer Matrix, die mindestens eine erste Matrixmaterial-Vorläuferkomponente und eine zweite Matrixmaterial-Vorläu - ferkomponente aufweist und zum Bilden der Matrix durch Vernetzen der Vorläuferkomponenten unter geeigneten Bedingungen in der Lage ist,
einen bioaktiven Faktor, der biologisch aktiv ist, um die Knochenbildung zwi - sehen zwei Wirbeln zu stimulieren, sowie zum Bewirken oder Unterstützen einer Wirbelsäulenfusion, wobei der bioaktive Faktor geeignet ist, bei der Vernetzung der Matrixmaterial-Vorläuferkomponenten lösbar in die Matrix eingebaut zu werden,
wobei der bioaktive Faktor PTH oder ein PTH aufweisendes Peptid ist, und
wobei ferner PTH oder das PTH aufweisende Peptid der einzige zugesetzte bioaktive Faktor ist, der zum Stimulieren der Knochenbildung zwischen zwei Wirbeln sowie zum Bewirken oder Unterstützen einer Wirbelsäulenfusion wirksam ist.

2. Pharmazeutische Formulierung nach Anspruch 1,
wobei die Zusammensetzung zum Bilden einer Matrix und der bioaktive Faktor als flüssige Komponenten vorliegen.

3. Pharmazeutische Formulierung nach Anspruch 1 oder 2,
die ferner ein körniges Material, vorzugsweise eine biologisch abbaubare keramische Verbindung, aufweist.

4. Pharmazeutische Formulierung nach einem der Ansprüche 1 bis 3,
wobei das PTH aufweisende Peptid ein PTH-Fusionspeptid ist, das mindes - tens zwei Domänen aufweist, wobei die erste Domäne PTH aufweist und die zweite Domäne eine vernetzbare Substratdomäne aufweist.

5. Pharmazeutische Formulierung nach Anspruch 4,
wobei die zweite Domäne des PTH-Fusionspeptids eine Transglutaminase-Substratdomäne, vorzugsweise eine Faktor XIIIa-Substratdomäne, aufweist.

6. Pharmazeutische Formulierung nach Anspruch 4 oder 5,
wobei das PTH-Fusionspeptid ferner eine enzymatische oder hydrolytischen Abbaustelle zwischen der ersten und der zweiten Domäne aufweist.

7. Pharmazeutische Formulierung nach Anspruch 6,
wobei die Abbaustelle ein Substrat für Plasmin ist.

8. Pharmazeutische Formulierung nach einem der Ansprüche 1 bis 7,
wobei das PTH aufweisende Peptid TGpl-PTH₁₋₃₄ ist.

9. Pharmazeutische Formulierung nach einem der Ansprüche 1 bis 7,
wobei die Matrix Fibrin ist und die Zusammensetzung zum Bilden der Matrix Fibrinogen, Thrombin und eine Calciumquelle aufweist.

10. Pharmazeutische Formulierung nach Anspruch 9,
wobei die Zusammensetzung zum Bilden der Fibrin-Matrix (i) Fibrinogen und Thrombin als erste und zweite Matrixmaterial-Vorläuferkomponente und optional Faktor Xllla sowie (ii) PTH in einem Bereich zwischen 0,01 bis 1 mg PTH/ml Fibrin-Matrix, vorzugsweise zwischen 0,2 bis 0,7 mg PTH/ml Fibrin-Matrix und in am meisten bevorzugter Weise 0,4 mg PTH/ml Fibrin-Matrix aufweist.

11. Pharmazeutische Formulierung nach einem der Ansprüche 1 bis 8,
wobei die Matrix eine synthetische Matrix oder eine gemischte synthetische/ natürliche Matrix ist, und wobei die Zusammensetzung zum Bilden der Ma - trix eine Matrixmaterial-Vorläuferkomponente mit starken nukleophilen Gruppen oder Bindungen sowie eine Matrixmaterial-Vorläuferkomponente mit starken elektrophilen Gruppen oder Bindungen aufweist.

12. Pharmazeutische Formulierung nach einem der Ansprüche 1 bis 11,
wobei es sich bei der Behandlung um eine Zwischenwirbel-Wirbelsäulenfusion handelt.

13. Kit zur Verwendung in einem Wirbelsäulenfusion-Behandlungsverfahren, aufweisend eine pharmazeutische Formulierung nach einem der Ansprüche 1 bis 12,
wobei mindestens eine der Matrixmaterial-Vorläuferkomponenten, die zum Bilden der Matrix durch Vernetzung der Vorläuferkomponenten unter geeig - neten Bedingungen in der Lage ist, getrennt von den übrigen Matrixmate - rial-Vorläuferkomponenten vorliegt.

14. Kit nach Anspruch 13,
wobei die erste Matrixmaterial-Vorläuferkomponente als erste Vorläuferiösung in einem ersten Behälter vorhanden ist, die zweite Matrixmaterial-Vor - läuferkomponente als zweite Vorläuferlösung in einem zweiten Behälter vor - handen ist und PTH oder das PTH aufweisende Peptid in der ersten oder der zweiten Vorläuferlösung vorhanden ist.

15. Kit nach Anspruch 13 oder 14,
wobei der Kit eine Wirbelsäulenfusionsvorrichtung, vorzugsweise einen Zwi - schenwirbel-Wirbelsäulenfusionskäfig, enthält.

16. Pharmazeutisches Produkt zur Verwendung in einem Wirbelsäulenfusions - verfahren, aufweisend eine Matrix und PTH oder ein PTH enthaltendes Peptid, das lösbar in die Matrix eingebaut ist, wobei das pharmazeutische Produkt durch Vernetzung der pharmazeutischen Formulierung nach einem der Ansprüche 1 bis 12 gebildet werden kann oder unter Verwendung des Kits nach einem der Ansprüche 13 bis 15 gebildet werden kann.

17. Zwischenwirbel-Wirbelsäulenfusionskäfig, der eine pharmazeutische Formu - lierung nach einem der Ansprüche 1 bis 12 enthält oder ein pharmazeuti - sches Produkt nach Anspruch 16 enthält.

18. Verfahren zum Bilden eines pharmazeutischen Produkts nach Anspruch 16, mit folgenden Schritten:
Bereitstellen einer ersten Matrixmaterial-Vorläuferkomponente,
Bereitstellen einer zweiten Matrixmaterial-Vorläuferkomponente,
Bereitstellen von PTH oder eines PTH aufweisenden Peptids, und
Mischen der ersten und der zweiten Matrixmaterial-Vorläuferkomponente und PTH oder des PTH aufweisenden Peptids, vorzugsweise unter physio - logischen Bedingungen, um ein Matrixmaterial durch Vernetzung der ersten und zweiten Matrixmaterial-Vorläuferkomponente zu bilden, wobei PTH oder das PTH aufweisende Peptid lösbar in das Matrixmaterial eingebaut wird.

19. Verfahren nach Anspruch 18, weiterhin aufweisend:
Bereitstellen eines körnigen Materials,
Benetzen des körnigen Materials, und
Mischen des benetzten körnigen Materials und der Mischung aus der ersten und der zweiten Matrixmaterial-Vorläuferkomponente und PTH oder dem PTH aufweisenden Peptid.

## Revendications

1. Formulation pharmaceutique pour son utilisation dans un procédé de spondylodèse, comprenant :
une composition destinée à la formation d'une matrice, comprenant au moins un premier composant précurseur de la matière matricielle et un second composant précurseur de la matière matricielle, capable de former la matrice par réticulation des composants précurseurs dans des conditions appropriées ;
un facteur bioactif qui est actif du point de vue biologique pour stimuler une formation osseuse entre deux vertèbres et pour mettre en œuvre une spondylodèse ou apporter son soutien à cette dernière, le facteur bioactif étant approprié pour être incorporé de manière amovible dans la matrice au cours de la réticulation des composants précurseurs de la matière matricielle ;
dans laquelle le facteur bioactif est la PTH ou un peptide comprenant de la PTH ; et en outre
dans laquelle la PTH ou le peptide comprenant de la PTH représente le seul facteur bioactif ajouté qui est efficace pour stimuler la formation osseuse qui est efficace pour stimuler une formation osseuse entre deux vertèbres et pour mettre en œuvre une spondylodèse ou apporter son soutien à cette dernière.

2. Formulation pharmaceutique selon la revendication 1, dans laquelle la formulation destinée à la formation d'une matrice et le facteur bioactif sont fournis sous la forme de composants liquides.

3. Formulation pharmaceutique selon la revendication 1 ou 2, comprenant en outre une matière granulaire, de préférence un composé céramique biodégradable.

4. Formulation pharmaceutique selon l'une quelconque des revendications 1 à 3, dans laquelle le peptide comprenant de la PTH représente un peptide de fusion à base de PTH comprenant au moins deux domaines, le premier domaine comprenant de la PTH et le second domaine comprenant un domaine de substrat réticulable.

5. Formulation pharmaceutique selon la revendication 4, dans laquelle le second domaine du peptide de fusion à base de PTH comprend un domaine de substrat de transglutaminase, de préférence un domaine de substrat du facteur xIIIa.

6. Formulation pharmaceutique selon la revendication 4 ou 5, dans laquelle le peptide de fusion à base de PTH comprend en outre un site de dégradation enzymatique ou hydrolytique entre le premier et le second domaine.

7. Formulation pharmaceutique selon la revendication 6, dans laquelle le site de dégradation représente un substrat pour de la plasmine.

8. Formulation pharmaceutique selon l'une quelconque des revendications 1 à 7, dans laquelle le peptide comprenant de la PTH est TGpl-PTH₁₋₃₄.

9. Formulation pharmaceutique selon l'une quelconque des revendications 1 à 7, dans laquelle la matrice représente de la fibrine et la composition destinée à la formation de la matrice comprend du fibrinogène, de la thrombine et une source de calcium.

10. Formulation pharmaceutique selon la revendication 9, dans laquelle la composition destinée à la formation de la matrice de fibrine comprend (i) du fibrinogène et de la thrombine à titre du premier et du second composant précurseur de la matière matricielle, et de manière facultative le facteur Xllla, et (ii) de la PTH dans une plage entre 0,01 et 1 mg de PTH/ml de matrice de fibrine, de préférence entre 0,2 et 0,7 mg de PTH/ml de matrice de fibrine, de manière de loin préférée 0,4 mg de PTH/ml de matrice de fibrine.

11. Formulation pharmaceutique selon l'une quelconque des revendications 1 à 8, dans laquelle la matrice est une matrice synthétique ou une matrice synthétique/naturelle mixte, et la composition destinée à la formation de la matrice comprend un composant précurseur de la matière matricielle possédant des liaisons ou des groupes nucléophiles forts, et un composant précurseur de la matière matricielle possédant des liaisons ou des groupes électrophiles forts.

12. Formulation pharmaceutique selon l'une quelconque des revendications 1 à 11, dans laquelle le traitement est une spondylodèse intersomatique.

13. Kit à utiliser dans un procédé de traitement de spondylodèse comprenant une formulation pharmaceutique selon l'une quelconque des revendications 1 à 12, dans lequel au moins un des composants précurseurs de la matière matricielle capables de former la matrice par réticulation des composants précurseurs dans des conditions appropriées est fourni séparément par rapport aux composants précurseurs restants de la matière matricielle.

14. Kit selon la revendication 13, dans lequel le premier composant précurseur de la matière matricielle est fourni sous la forme d'une première solution précurseur dans un premier récipient, le second composant précurseur de la matière matricielle est fourni sous la forme d'une seconde solution précurseur dans un second récipient et la PTH ou le peptide comprenant de la PTH est fourni dans la première ou dans la seconde solution précurseur.

15. Kit selon la revendication 13 ou 14, contenant en outre un dispositif de spondylodèse, de préférence une cage de spondylodèse intersomatique.

16. Produit pharmaceutique pour son utilisation dans un procédé de spondylodèse comprenant une matrice et de la PTH ou un peptide contenant de la PTH incorporé de manière amovible dans la matrice, dans lequel le produit pharmaceutique peut être obtenu par réticulation de la formulation pharmaceutique revendiquée dans l'une quelconque des revendications 1 à 12, ou bien peut être obtenu en utilisant le kit tel que revendiqué dans l'une quelconque des revendications 13 à 15.

17. Cage de spondylodèse intersomatique contenant une formulation pharmaceutique telle que revendiquée dans l'une quelconque des revendications 1 à 12, ou contenant un produit pharmaceutique tel que revendiqué à la revendication 16.

18. Procédé pour la formation d'un produit pharmaceutique tel que revendiqué à la revendication 16, comprenant le fait de :
procurer un premier composant précurseur de la matière matricielle ;
procurer un second composant précurseur de la matière matricielle ;
procurer de la PTH ou un peptide comprenant de la PTH ; et
mélanger le premier et le second composant précurseur de la matière matricielle et la PTH ou le peptide comprenant de la PTH, de préférence dans des conditions physiologiques, pour obtenir une matière matricielle par réticulation du premier et du second composant précurseur de la matière matricielle, la matière matricielle possédant de la PTH ou le peptide comprenant de la PTH à l'état incorporé de manière amovible.

19. Procédé selon la revendication 18, comprenant en outre le fait de :
procurer une matière granulaire ;
humidifier la matière granulaire ; et
mélanger la matière granulaire humidifiée et le mélange du premier et du second composant précurseur de la matière matricielle et de la PTH ou du peptide comprenant de la PTH.
